# EUROPEAN PATENT APPLICATION

(11) **EP 0 711 541 A1**
(43) Date of publication of application: **15.05.1996**
(21) Application number: 94308335.2
(22) Date of filing: 11.11.1994
(51) Int. Cl.: A61K 7/06

(54) **Use of glucose blockers for inhibiting hair growth**

(71) Applicant: Leveen, Harry H., Charleston, SC 29407 (US)
(72) Inventor: Leveen, Harry H., Charleston, SC 29407 (US)
(74) Representative: W.P. Thompson & Co.

(57) **Abstract**

A composition (e.g. a solution) for inhibiting hair growth in mammals which contains as the active ingredient a compound which block glucose transfer across the membranes of the cells of hair follicles. The compound is used in a method of inhibiting hair growth in mammals including topically applying the compound to any area of unwanted hair e.g. on a daily basis. A preferred embodiment of the invention uses phloretin as a glucose blocking compound. Other embodiments of the invention include steviol, cytochlasin B, 3,3'diallylstilbesterol and 3,3'-di(2-chlorallyl) stilbesterol. The blocking compounds are suspended in solutions of suitable solvents.

## Description

The present invention is directed to a method and a composition for inhibiting hair growth. More particularly, it is directed toward a method employing a glucose blocking compound, e.g. phloretin which is topically administered to areas of unwanted hair growth.

Resting mammalian cells are supplied glucose and oxygen by the circulation of blood. The cells utilize the oxygen to convert absorbed glucose into carbon dioxide and water by oxidation. This oxidation process yields energy which is used to synthesize adenosine triphosphate (ATP) which is a high energy compound. Energy contained in the ATP is rapidly released by the liberation of two phosphate groups and becomes available for carrying out biochemical reactions required by cell functions. Chemical reactions requiring a thermodynamic expenditure of energy also use the degradation of ATP as a source of energy.

Rapidly growing cells require energy not only for normal cell functions but also for the synthesis of cellular mass. Such cells utilize glucose as a building block for the formation of new protein and nucleic acids. Rapidly dividing cells utilize aerobic glycolysis, converting glucose to lactic acid instead of carbon dioxide and water. Growing fetal cells and lymphocytes which are stimulated by mitogens have been studied and found to utilize this process. Roos D, Loos JA, "Changes in the Carbohydrate Metabolism of Mitigenically Stimulated Human Peripheral Lymphocytes." Experimental Cell Research (1973) 77:127-135.

It is known that the cells of hair follicles behave like most other normal mammalian cells in their use of carbohydrates. Adachi K., "The Metabolism and Control Mechanism of Human Hair Follicles." Curr. Probl. Derm., p. 37-38, (1973) Karger, Basel. However, the cells of hair follicles are exceptional as being among the fastest growing cells in the human body. Weistein, G.D., "Cell Proliferation Kinetics in the Human Hair Root." J. Invest. Derm., 74:43-46 (1980). Accordingly, hair growth requires rapid utilization of glucose by the process of aerobic glycolysis. It has been found that up to 90% of the glucose metabolism in hair follicle cells is accomplished by aerobic glycolysis. Philpott, M. P., Kealey, T. "Metabolic Studies on Isolated Hair Follicles: Hair Follicles Engage in Aerobic Glycolysis and Do Not Demonstrate The Glucose Fatty Acid Cycle." J. Invest. Derm. (1991) 96:875-79. Therefore, hair growth may be inhibited by depriving hair follicle cells of the supply of glucose since hair growth is dependent upon the rapid metabolization of glucose.

Circulation of blood to hair follicle cells provides a constant source of glucose. Blood contains close to 100 mg. of glucose/dl and even higher levels after meals. Because the circulation of blood to the hair follicles is excellent, it is not possible to prevent glucose from reaching the hair follicle cells.

Glucose is water soluble and impermeable to the cell wall. It can only be transported across the cell wall at specific small islands of hydrated protein on the cell surface known as receptor sites. The receptor sites on the cell's surface contain specific receptor proteins and utilize enzyme systems to effect the transfer of glucose into the cell by the process of facilitated diffusion. Thus, if the receptor sites are disabled, glucose cannot be adsorbed by the cell despite the constant supply to the cell surface.

Several non-metabolizable sugars, such as 2-methoxy glucose can block the transfer of glucose. However, their use poisons the enzyme systems. Alternatively, glucose blocking compounds that bond to receptor sites may be used to prevent metabolization of glucose.

Glucose blocking compounds are known, with the earliest having been isolated at the end of the 19th century. The majority of these substances are phenolic compounds of varying complexity some of which are easily synthesized in the laboratory. Le Fevre, P. "Sugar Transport in the Red Blood Cell: Structure-Activity Relationships in Substances and Antagonists." Pharm. Rev. (1961) 13:39-70.

Phloridzin is a phenolic compound which functions as a glucoside, blocking receptor sites on cell walls. Phloridzin is a natural plant hormone present in apple root bark, Merck Index 10:7211, 1983, and in the seeds of immature apples. Nature, 158:663, 1946. Intravenous injection of phloridzin causes glucosuria in animals and humans because it blocks absorption of glucose by renal tubular cells, thereby preventing absorption of glucose from the glomerular filtrate. Diedrich, D.F. "The Comparative Effects of Some Phloridzin Analogs on the Renal Absorption of Glucose." Biochem. Biophys. Acta. (1963) 71:688-700; Wen, S.F. "Effect of Phloridzin on Renal Glucose and Phosphate Transport in the Dog." Clin. Sci. (1979) 57:367-374. The physiological effects of Phloridzin and its aglucone phloretin have been extensively studied. McKee, F. W., Hawkins, W. B. "Phlorhizin Diabetes." Physiol. Rev. (1927) 25:255; Lotspeich, W. D. "Phlorizin and the Cellular Transport of Glucose." Harvey Lect. (1961) 56:63-91.

### SUMMARY OF THE INVENTION

Phloridzin is known to be very effective in blocking glucose transfer into the renal tubular cells and the cells of the intestinal mucosa where the glucose transport is intimately related to sodium transport. Such glucose transport is known to be sodium dependent. Phloretin, the aglucone of phloridzin, on the other hand has a diminished effect on this type of glucose transfer and parenteral administration may fail to cause glucosuria in animals and man. By contrast, phloretin is much more active on non-sodium dependent transfer of glucose, since non-sodium dependent glucose transport is the normal transfer mechanism of most cells other than those of the renal tubule and intestinal mucosa. This type of transfer has been extensively studied in the red blood cell where precise measurements can be made. Le Fevre, supra. Our studies have shown that glucose transport into the follicular cells which form hair is identical to that found in red blood cells. For this reason, phloretin is much more effective than phloridzin.

Accordingly, it is an object of the present invention to provide a method for inhibiting hair growth.

It is a further object of the invention to provide a topically applied composition (e.g. a solution) for preventing glucose from entering the cells of hair follicles to arrest cellular growth.

Briefly stated, the present invention provides a composition for inhibiting hair growth in mammals which contains as the active ingredient a compound which blocks glucose transfer across the membranes of the cells of hair follicles. The compound is used in a method of inhibiting hair growth in mammals including topically applying the compound e.g. as a solution to any area of unwanted hair on a daily basis. A preferred embodiment of the invention uses phloretin as a glucose blocking compound. Other embodiments of the invention include steviol, cytochlasin B, 3,3'diallylstilbesterol and 3,3'-di(2-chlorallyl) stilbesterol. The blocking compounds are suspended in solutions of suitable solvents which may include propylene glycol and ethyl alcohol.

The invention also includes a method of inhibiting hair growth on the skin of a mammal by administering topically to the skin a preparation containing a glucose blocking compound.

In accordance with the present invention hair growth is inhibited by topically applying a glucose blocking compound. Phloretin is a preferred glucose blocking compound. Phloretin is not soluble in aqueous solution, but is soluble in some solvents including propylene glycol, ethyl alcohol, dimethlsufoxide (DMSO) and other solvents. Phloretin prevents the entrance of glucose into cells by blocking the glucose transfer sites on the cell membrane. Phloretin is a complex diphenolic compound, which is the aglucon of phloridzin.

The following example is intended to be illustrative, but not limitative of the present invention.

### EXAMPLE

Phloretin is dissolved in a solution containing 20% propylene glycol and 80% ethyl alcohol to yield a solution having a final concentration of 5-7.5% phloretin. The resulting solution is applied topically to any area of unwanted hair growth on a daily basis.

Another embodiment of the present invention utilizes cytochlasin B which is an effective glucose blocking compound that firmly attaches itself to the glucose receptor protein of a single receptor. The uptake of radioactive cytochlasin B is used to determine the number of receptor sites present on a cells surface. Because cytochalasin B is less soluble in propylene glycol and ethyl alcohol some DMSO is added to these solvents.

Still other embodiments of the present invention include effective glucose blocking compounds such as steviol, 3,3'diallylstilbesterol, and 3,3'-di(2-chlorallyl) stilbesterol.

## Claims

1. A method of inhibiting hair growth on the skin of a mammal by administering topically to the skin a preparation containing a glucose blocking compound.

2. A method as claimed in claim 1, wherein said glucose blocking compound is selected from the group consisting of phloretin, steviol, cytochlasin B, 3,3'diallylstilbesterol and 3,3'-di(2-chlorallyl) stilbesterol.

3. A method as claimed in claim 1 or 2, wherein said preparation is administered at a rate of at least one application per day.

4. A method as claimed in any one of the preceding claims, wherein said glucose blocking compound is phloretin and said preparation further comprises propylene glycol and ethyl alcohol.

5. A composition capable of inhibiting hair growth in a mammal comprising an amount of a glucose blocking compound effective to inhibit hair growth.

6. A composition as claimed in claim 5, wherein said glucose blocking compound is selected from the group consisting of phloretin, steviol, cytochlasin B, 3,3'diallylstilbesterol and 3,3'-di(2-chlorallyl) stilbesterol.

7. A composition as claimed in claims 5 or 6, further comprising propylene glycol and ethyl alcohol.

8. A composition capable of inhibiting hair growth comprising phloretin in a concentration of 5 to 7 ½% in a solvent mixture including 20% propylene glycol and 80% ethyl alcohol
